# EUROPÄISCHE PATENTSCHRIFT

(11) **EP 3 527 653 B1**
(45) Veröffentlichungstag und Bekanntmachung des Hinweises auf die Patenterteilung: **19.01.2022**
(21) Anmeldenummer: 18157634.9
(22) Anmeldetag: 20.02.2018
(51) Int. Cl.: C12M 1/00, C12M 1/34

(54) **EXPOSITIONSANLAGE UND VERFAHREN ZUM KONTINUIERLICHEN BEGASEN WENIGSTENS EINER ZELLKULTUR**
EXPOSURE INSTALLATION AND METHOD FOR THE CONTINUOUS GASSING OF AT LEAST ONE CELL CULTURE
SYSTÈME D'EXPOSITION ET PROCÉDÉ DE GAZAGE CONTINU D'AU MOINS UNE CULTURE CELLULAIRE

(43) Veröffentlichungstag der Anmeldung: 21.08.2019
(73) Patentinhaber: Fritz Egger GmbH & Co. OG, 6380 St. Johann in Tirol (AT); Medizinische Universität Innsbruck, 6020 Innsbruck (AT); Steinlechner, Reinhold, 6200 Jenbach (AT)
(72) Erfinder: Gostner, Johanna, 6175 Kematen in Tirol (AT); Zeisler, Johannes, 6322 Kirchbichl (AT); Steinlechner, Reinhold, 6200 Jenbach (AT)
(74) Vertreter: Cohausz & Florack

(56) Entgegenhaltungen:
- WO-A2-03/076599
- DE-A1- 10 128 810
- DE-A1-102008 056 686
- US-B1- 9 588 105
- JOHANNA M. GOSTNER ET AL: "Cellular reactions to long-term volatile organic compound (VOC) exposures", SCIENTIFIC REPORTS, Bd. 6, Nr. 1, 31. Dezember 2016 (2016-12-31), XP55464935, DOI: 10.1038/srep37842
- DATABASE WPI Week 201469 Thomson Scientific, London, GB; AN 2014-T06062 XP002783963, & CN 203 754 722 U (BEIJING HUIRONGHE SCI & TECHNOLOGY CO) 6. August 2014 (2014-08-06)
- DATABASE WPI Week 201147 Thomson Scientific, London, GB; AN 2011-H43561 XP002783964, & CN 201 842 846 U (TIANJIN DEV ZONE HEPU IND&TRADE CO LTD) 25. Mai 2011 (2011-05-25)

## Beschreibung

Die Erfindung betrifft eine Expositionsanlage zum kontinuierlichen Begasen wenigstens einer Zellkultur mit wenigstens einem Testgas, mit wenigstens einer Probenaufnahme zum Bereithalten der wenigstens einen Zellkultur während des Begasens und mit wenigstens einer Konditioniereinrichtung zur Konditionierung des wenigstens einen Testgases, wobei die Konditioniereinrichtung wenigstens eine Trägergaszuführung, wenigstens eine Testkomponentenzugabe und wenigstens eine Testgasbefeuchtung aufweist und wobei die wenigstens eine Testgasbefeuchtung wenigstens einen Wasserbehälter und/oder die Testkomponentenzugabe wenigstens einen Standardbehälter zum Bereithalten wenigstens eines die wenigstens eine zu dosierende Testkomponente aufweisenden Standards aufweist und wobei dem Standardbehälter wenigstens eine Druckversorgung zum Beaufschlagen des zu dosierenden Standards im Standardbehälter mit einem vorbestimmten Druck zugeordnet ist. Ferner betrifft die Erfindung ein Verfahren zum Betreiben einer Expositionsanlage zum kontinuierlichen Begasen wenigstens einer Zellkultur in wenigstens einer Probenaufnahme mit wenigstens einem Testgas.

Expositionsanlagen, mit denen Zellkulturen einer kontrollierten Gasatmosphäre ausgesetzt werden können, sind in unterschiedlicher Ausprägung bekannt. Die Expositionsanlagen dienen dabei der Untersuchung der Auswirkungen von bestimmten Stoffen auf den menschlichen Organismus. Die Stoffe, die mit entsprechenden Expositionsanlagen getestet werden, sind typischerweise solche, die zumindest potentiell toxisch oder krebserregend eingestuft werden. Des Weiteren handelt es sich insbesondere um solche Stoffe, die über die Atemwege aufgenommen werden, wie etwa flüchtige organische Verbindungen (Volatile Organic Compounds - VOC). Um die Aufnahme der Stoffe über die Atemwege zu simulieren, werden die Zellkulturen mit einem Testgas mit einer bestimmten Konzentration der zu testenden Komponente überströmt, wobei als Zellkuturen beispielsweise Lungenepithelzellen oder Immunzellen in Frage kommen.

In letzter Zeit werden verstärkt die Auswirkungen von flüchtigen organischen Verbindungen, insbesondere von Formaldehyd, in Wohnräumen und Arbeitsräumen diskutiert, die aus Bauwerken, Möbeln und dergleichen emittiert werden. Gleichzeitig sind jedoch die Langzeitauswirkungen solcher Komponenten auf die Gesundheit sowie die Ausbildung von Asthma und Allergien noch weitgehend ungeklärt, und zwar insbesondere im Wechselspiel mit weiteren Komponenten. Aber erst wenn diese Erkenntnisse gewonnen wurden, können verlässliche Grenzwerte für Wohn- und Arbeitsbereiche festgelegt werden.

Viele der bekannten Expositionsanlagen erlauben Tests nur über einen kurzen Zeitraum, da die Zellkulturen den Testbedingungen nur für einen begrenzten Zeitraum widerstehen. Mit der Zeit verändern sich die Zellkulturen, so dass dann keine übertragbaren Erkenntnisse mehr gewonnen werden können. Für Langzeittests müssen die Zellkulturen in den Expositionsanlagen über lange Zeit mit einem Testgas überströmt werden, dessen Zusammensetzung immer konstant bleibt. Schon geringe Abweichungen bezogen auf die Konzentration der zu testenden Komponente und/oder der Feuchtigkeit des Testgases kann zu einer nachhaltigen Beeinträchtigung der Zellkulturen und/oder der Messergebnisse führen. Dies begründet sich auch darin, dass während der Langzeittests nicht lediglich epitheliale Parameter, sondern auch Stoffwechselprozesse und Stoffwechselmengen, bewertet werden. Dabei kann die Feuchtigkeit des Testgases zu unterschiedlichen Problemen führen. Ist die Feuchtigkeit auch nur zeitweise zu gering, können die Zellkulturen austrocknen. Ist die Feuchtigkeit zu hoch, kann es zu Kondensation kommen, durch die dem Testgas auch erhebliche Anteile der zu testenden Komponenten entzogen werden können, was die Messung direkt beeinträchtigt.

Eine Expositionsanlage und ein Verfahren zu deren Betrieb ist in Gostner J. M., Zeisler J. et al. "Cellular reactions to long-term volatile organic compound (VOC) exposures" Scientific Reports 6, 37842, doi: 10.1038/srep37842, 2016, beschrieben. Für die Einstellung der Testgaszusammensetzung wurde hier auf eine separate Testgasbefeuchtung, eine Testkomponentenzugabe und eine separate Trägergaszuführung zurückgegriffen, wobei die Feuchtigkeit, die Testkomponente und das Trägergas gemischt und gemeinsam als Testgas der Probenaufnahme in einer temperierten Expositionskammer zugeführt werden. Dabei weist die Testgasbefeuchtung einen temperierten Wasserbehälter und die Testkomponentenzugabe einen mit Druck beaufschlagten Standardbehälter auf. Durch den temperierten Wasserbehälter werden Luft und Kohlendioxid (CO₂) zum Befeuchten hindurchgeleitet. Die Massenströme an Luft und Kohlendioxid werden dabei über Massendurchflussregler geregelt. Dem mit Druck beaufschlagten Standardbehälter wird ein vorgegebener Massenstrom entnommen, der über einen Massendurchflussregler geregelt und anschließend verdampft wird. Bei dem Standard handelt es sich vorzugsweise um eine Flüssigkeit bzw. Lösung, die einen bestimmten, vorgegebenen Anteil einer Testkomponente aufweist. Die Testkomponente kann im Standard gelöst oder absorbiert sein. Durch das Verdampfen einer bestimmten Menge des Standards kann eine bestimmte Mange an Testkomponente zum Begasen der Zellkulturen bereitgestellt werden. Der Standard könnte prinzipiell auch die Testkomponente in Reinform sein. Die Dosierung ist aufgrund der geringen benötigten Mengen an Testkomponente jedoch einfacher und genauer, wenn die Testkomponente nur einen kleinen Teil des Standards ausmacht. Zudem kann der Standard bedarfsweise auch mehrere Testkomponenten aufweisen, mit denen die Zellkulturen zeitgleich begast werden sollen. Eine exakte, dauerhafte Einstellung der Testgaszusammensetzung hat sich aber auch hier als noch nicht zufriedenstellend gelöst dargestellt.

Mithin liegt der vorliegenden Erfindung die Aufgabe zugrunde, die Expositionsanlage und das Verfahren jeweils der eingangs genannten und zuvor näher beschriebenen Art derart auszugestalten und weiterzubilden, zuverlässiger Langzeitmessungen mit Expositionsanlagen durchführen zu können.

Diese Aufgabe ist bei einer Expositionsanlage nach dem Oberbegriff des Anspruchs 1 dadurch gelöst, dass dem Wasserbehälter wenigstens eine Druckversorgung zum Beaufschlagen des zum Testgas zu dosierenden Wassers im Wasserbehälter mit einem vorbestimmten Druck vorgesehen ist, dass dem Wasserbehälter eine Wasserkapillare zum Durchleiten des zu dosierenden und mit dem vorbestimmten Druck beaufschlagten Wassers nachgeschaltet ist und dass der Wasserkapillare ein Verdampfer zum Verdampfen des die Wasserkapillare passierenden Wassers nachgeschaltet ist.

Die genannte Aufgabe ist zudem bei einer Expositionsanlage nach dem Oberbegriff des Anspruch 2 dadurch gelöst, dass dem Standardbehälter eine Flüssigkeitskapillare zum Durchleiten des zu dosierenden und mit dem vorbestimmten Druck beaufschlagten Standards nachgeschaltet ist und dass der Flüssigkeitskapillare ein Verdampfer zum Verdampfen des die Flüssigkeitskapillare passierenden Standards nachgeschaltet ist.

Des Weiteren ist die zuvor genannte Aufgabe gemäß Anspruch 15 gelöst durch ein Verfahren zum Betreiben einer Expositionsanlage, zum kontinuierlichen Begasen wenigstens einer Zellkultur in wenigstens einer Probenaufnahme mit wenigstens einem Testgas, insbesondere nach einem der Ansprüche 1 bis 14,
- bei dem in wenigstens einer Konditioniereinrichtung das wenigstens eine Testgas zum Begasen der wenigstens einen Zellkultur konditioniert wird,
- bei dem in der wenigstens einen Konditioniereinrichtung, insbesondere wenigstens einer Testgasbefeuchtung, zu dosierendes Wasser aus wenigstens einem druckbeaufschlagten Wasserbehälter mit einem vorbestimmten Druck einer Wasserkapillare zugeführt und das Wasser nach dem Verlassen der Wasserkapillare verdampft wird und/oder
- bei dem in der wenigstens einen Konditioniereinrichtung, insbesondere wenigstens einer Testkomponentenzugabe, zu dosierender Standard aus wenigstens einem druckbeaufschlagten Standardbehälter mit einem vorbestimmten Druck einer Flüssigkeitskapillare zugeführt und der Standard nach dem Verlassen bei der Flüssigkeitskapillare verdampft wird.

Die Erfindung hat erkannt, dass eine gleichmäßigere und genauere Dosierung der Feuchtigkeit und der Testkomponente bzw. des Standards erfolgen kann, wenn zugunsten einer Kapillare auf den Massendurchflussregler verzichtet wird. Massendurchflussregler sind typischerweise besonders geeignet, einen Massenstrom dauerhaft sehr präzise zu regeln. Bei den vorliegenden sehr geringen Massenströmungen ist dies jedoch nur bedingt möglich. In diesem Zusammenhang wirkt es sich offenbar als nachteilig aus, dass das Trägergas nach dem Passieren der Massendurchflussregler noch durch eine Waschflasche geleitet wird. Die Feuchtigkeitsaufnahme scheint hier trotz einer Temperierung der Waschflasche nicht exakt und konstant zu erfolgen. Dies mag mit Schwankungen des Massenstroms an Trägergas zusammenhängen, dürfte aber auch maßgeblich von Temperaturschwankungen in der Waschflasche bedingt werden.

Diese möglichen Probleme können ausgeräumt werden, wenn der Wasserbehälter unter einen konstanten Druck gesetzt wird, was durch marktverfügbare Reduzierventile problemlos möglich ist, und das Wasser so mit einem konstanten Druck durch eine Wasserkapillare gedrückt wird. Aufgrund des hohen Strömungswiderstands des Wassers in der Wasserkapillare ist der Durchfluss an Wasser durch die Wasserkapillare direkt von dem Druck des Wassers am Eintritt in die Wasserkapillare abhängig. Dies wird genutzt um einen konstanten und genau einzustellenden Massenstrom an Wasser zu erzeugen. Um das Wasser als Feuchtigkeit in die Gasphase zu bringen, sind zudem keine Waschflaschen mehr erforderlich. Dies kann viel zuverlässiger durch einen Verdampfer erfolgen, der problemlos eine vollständige Verdampfung des Wassers sicherstellt.

Was die Dosierung der Testkomponente betrifft, wurde diese bereits im Stand der Technik der Standardbehälter mit Druck beaufschlagt. Der aus dem Standardbehälter herausgedrückte Massenstrom an Standard wird damit durch einen Massendurchflussregler geregelt. Anschließend erfolgt die Verdampfung des Standards in einem Verdampfer. Es wird angenommen, dass die Verdampfung des Standards zu Druckschwankungen beiträgt, die auf den Massendurchflussregler zurückwirken und die entsprechende Regelung des Massenstroms an Standard beeinträchtigen.

Die Erfindung ermöglicht jedenfalls eine in höchstem Maße störungsfreie und genaue Einstellung der Testkomponente. Dazu wird über den unter einem bestimmten Druck stehenden Standardbehälter wie beim Wasserbehälter der Standard durch eine Flüssigkeitskapillare gedrückt und anschließend in einem Verdampfer vollständig verdampft. Auch hier ist es wie beim Wasser durch die Verwendung einer Flüssigkeitskapillare möglich, den Massenstrom über den Druck des Standards vor der Flüssigkeitskapillare sehr genau einzustellen.

In diesem Zusammenhang wird darauf hingewiesen, dass die Testkomponentenzugabe und die Testgasbefeuchtung auch unabhängig voneinander eingesetzt werden können. Sowohl die Testkomponentenzugabe als auch die Testgasbefeuchtung führen jede für sich zu den zuvor angesprochenen Vorteilen. Besonders bevorzugt ist es dabei jedoch, wenn Testkomponentenzugabe und die Testgasbefeuchtung wie beschrieben ausgestaltet werden, um dann die entsprechenden Vorteile kombinieren zu können.

Dabei kann es sich bei der Testkomponente um grundsätzlich sehr unterschiedliche Stoffe handeln. Die Erfindung ist auch nicht auf die Verwendung einer einzigen Testkomponente beschränkt. Es können durchaus auch mehrere Testkomponenten gleichzeitig für die Bildung eines Testgases herangezogen werden. Vorzugsweise wird dann ein Standard verwendet, in dem alle Testkomponenten im richtigen Verhältnis zueinander enthalten sind, um ein Testgas mit der gewünschten Testgaszusammensetzung zu erhalten. Es können aber auch verschiedene Standards in verschiedenen Standardbehältern vorgehalten werden. Dann kann die Dosierung jedes einzelnen Standards wie zuvor beschrieben erfolgen. Jeder Standard wird dann durch eine separate Flüssigkeitskapillare gedrückt und in einem separaten Verdampfer anschließend verdampft. Die einzelnen verdampften Standards können dann nachträglich in einem Mischer oder in mehreren Mischern zusammengeführt werden. Zwischen dem wenigstens einen Mischer und wenigstens einem Verdampfer kann bedarfsweise noch eine Dampfkapillare zum Puffern des Drucks und des Massenstroms vorgesehen sein.

Besonders bevorzugt handelt es sich bei der wenigstens einen Testkomponente um eine flüchtige organische Verbindungen (Volatile Organic Compounds - VOC), da Kenntnisse über deren Auswirkungen auf den menschlichen Organismus von besonderem Interesse sind. Dies gilt beispielsweise für Formaldehyd (CH₂O) und viele andere Testkomponenten.

Als Zellkulturen, deren Wechselwirkungen mit den Testkomponenten in der Expositionsanlage untersucht werden können, sind Epithelzellen in Monokultur oder in Kokultur mit anderen Zelltypen. Bei einer solchen Kokultur kann es sich bevorzugt um Lungenepithelzellen in Kokultur mit Immunzellen handeln, um die Aufnahme von potentiell bedenklichen Stoffen über die Atemwege zu simulieren.

Verfahrensmäßig erlaubt die Erfindung einen bevorzugten Betrieb einer Konditioniereinrichtung zur Konditionierung eines Testgases zum Begasen der wenigstens einen Zellkultur in der Expositionsanalage, in der zu dosierendes Wasser aus wenigstens einem durckbeaufschlagten Wasserbehälter mit einem vorbestimmten Druck einer Wasserkapillare zugeführt und das Wasser nach dem Verlassen der Wasserkapillare verdampft wird. Alternativ oder bevorzugt gleichzeitig kann der zu dosierende Standard aus wenigstens einem druckbeaufschlagten Standardbehälter mit einem vorbestimmten Druck einer Flüssigkeitskapillare zugeführt und der Standard nach dem Verlassen der Flüssigkeitskapillare verdampft werden.

Hierdurch werden bedarfsweise die bereits im Zusammenhang mit den entsprechenden Expositionsanlagen beschriebenen Vorteile erreicht.

Der besseren Verständlichkeit halber und zur Vermeidung unnötiger Wiederholungen werden nachfolgend die Expositionsanlage und das Verfahren zu deren Betrieb gemeinsam beschrieben, ohne jeweils im Einzelnen zwischen der Expositionsanlage und dem Verfahren zu unterscheiden. Für den Fachmann ergibt sich anhand des Kontextes, welches Merkmal jeweils im Hinblick auf die Expositionsanlage und des Verfahrens besonders bevorzugt ist.

Bei einer ersten besonders bevorzugten Ausgestaltung der Expositionsanlage weist die Druckversorgung des Wasserbehälters und/oder des Standardbehälters wenigstens einen Druckgasanschluss, insbesondere Druckluftanschluss, auf. So lässt sich einfach und dauerhaft ein konstanter Druck im Wasserbehälter und/oder Standardbehälter aufrechterhalten. Ohne einen entsprechenden Druckgasanschluss oder wenn der Druck im Druckgasanschluss nicht bereits durch andere Maßnahmen in hohem Maße konstant bereitgestellt und/oder einstellbar ist, bietet es sich an, wenn wenigstens eine Druckregelung zur Regelung des Drucks im Wasserbehälter und/oder im Standardbehälter vorgesehen ist. In einem apparativ einfachen Fall kann ein Reduzierventil in der Druckgasleitung vorgesehen werden.

Für eine genaue und konstante Dosierung von Feuchtigkeit und/oder des Standards bzw. der wenigstens einen Testkomponente hat es sich als zweckmäßig herausgestellt, wenn der Druck im Wasserbehälter und/oder im Standardbehälter zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, beträgt.

Um eine vollständige und zudem gleichmäßige Verdampfung des Wassers und/oder des Standards zu erreichen, kann dem Verdampfer zum Verdampfen des Wassers und/oder des Standards wenigstens eine Verdampferregelung zur Regelung der Temperatur des verdampften Wassers und/oder des verdampften Standards zugeordnet sein. Die Verdampferregelung kann alternativ oder zusätzlich auch die Temperatur des Verdampfers selbst regeln, wenn dies zweckdienlich ist und sich die Temperaturen von Verdampfer und verdampftem Waser bzw. Standard in der Praxis überhaupt nennenswert unterscheiden.

Zudem kann die Temperatur des verdampften Wassers und/oder Standards im Verdampfer vorzugsweise auf einen bestimmten Wert zwischen 100°C und 300°C, vorzugsweise zwischen 120°C und 200°C, insbesondere zwischen 130°C und 170°C, eingestellt bzw. geregelt werden. Alternativ oder zusätzlich kann auch der Druck des verdampften Wassers vor der Wasserdampfkapillare und/oder des verdampften Standards vor der Dampfkapillare auf einen bestimmten Wert zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, eingestellt bzw. geregelt werden. Dies ermöglicht ein vollständiges und gleichmäßiges Verdampfen des Wassers und/oder des Standards. Außerdem wird so sichergestellt, dass vor der Beaufschlagung der Zellkulturen mit dem Testgas keine unerwünschte Kondensation von Feuchtigkeit und/oder Standard auftritt.

Um eine möglichst homogene Vermischung von Feuchtigkeit, Testkomponente und/oder Trägergas zu erreichen, so dass ein möglichst homogenes Testgas zum Beaufschlagen der Zellkulturen erreicht wird, kann dem Verdampfer des Wassers, dem Verdampfer des Standards, der Wasserdampfkapillare und/oder der Dampfkapillare ein Mischer nachgeschaltet werden, bei dem es sich in besonders bevorzugter Ausgestaltung um einen statischen Mischer handeln kann. Dabei kann der Mischer sowohl zum Vermischen des verdampften Wassers und des verdampften Standards untereinander dienen als auch ergänzend oder gleichzeitig für das Vermischen des verdampften Wassers und/oder des verdampften Standards mit dem Trägergas genutzt werden. Das Vermischen der Einzelströme zum Testgas kann mithin durch einen einzigen Mischer oder durch eine Mehrzahl von Mischern, insbesondere statischen Mischern, bewerkstelligt werden.

Die Trägergaszuführung kann der Einfachheit halber wenigstens einen Gasanschluss, wenigstens einen Luftanschluss und/oder Kohlendioxidanschluss (CO2), aufweisen, um entsprechendes Gas zuzuführen. Dabei bietet es sich an, dem wenigstens einen Gasanschluss einen Massendurchflussregler (Mass Flow Controller) zum Regel des zugeführten Massenstroms des über den Gasanschluss zugeführten Gases, insbesondere Trägergases, nachzuschalten. Darüber hinaus ist für eine gleichmäßige Zuführung des Trägergases bevorzugt, wenn dem wenigstens einen Gasanschluss und/oder dem wenigstens einen Massendurchflussmesser eine Verteileinrichtung zum gleichmäßigen Aufteilen des Trägergases zum Begasen von unterschiedlichen Zellkulturen mit unterschiedlichen Testgaskonzentrationen nachgeschaltet ist. So muss nicht jeder einzelne Trägergasstrom über separate Massendurchflussmesser eingestellt werden. Hierzu bieten sich insbesondere kritische Düsen an, die jeweils eine sehr kleine Öffnung aufweisen. Die Öffnung ist bei allen kritischen Düsen gleich groß, so dass der Durchfluss über die kritischen Düsen in genau gleiche Anteile aufgeteilt werden kann. Es kann also beispielsweise eine Expositionsanlage bereitgestellt werden, die mehrere Zellboxen zum Begasen von Zellkulturen mit unterschiedlichen Testgaskonzentrationen sowie dementsprechend mehrere Testgasbefeuchtungen und Testkomponentenzugaben umfasst. Dagegen kann dann nur eine Trägergaszuführung mit einer Verteileinrichtung vorgesehen sein, die Trägergasströme für jede der Zellboxen bereitstellt.

Der wenigstens eine Massendurchflussregler regelt dabei vorzugsweise den Massenstrom des über den Gasanschluss zugeführten Kohlendioxids auf einen möglichst konstanten, vorbestimmten Wert zwischen 5 Nl/h (Normliter pro Stunde) und 100 Nl/h, vorzugsweise zwischen 10 Nl/h und 50 Nl/h, insbesondere zwischen15 Nl/h und 30 Nl/h. Alternativ oder zusätzlich kann der wenigstens eine Massendurchflussregler den Massenstrom der zugeführten Luft auf einen möglichst konstanten, vorbestimmten Wert zwischen 100 Nl/h und 1200 Nl/h, vorzugsweise zwischen 200 Nl/h und 800 Nl/h, insbesondere zwischen 300 Nl/h und 500 Nl/h, regeln. Beides führt zu einer angemessenen Trägergasmenge zum Beaufschlagen der Zellkulturen ohne diese rein mechanisch zu schädigen bzw. zu stressen. Zur besseren Vergleichbarkeit von Gasmengen bei unterschiedlichen Drücken und/oder Temperaturen, werden diese technisch auf Normbedingungen umgerechnet. Die Angabe Nl stellt also eine Volumenangabe unter Normbedingungen dar, wobei die Normbedingungen (technischer Normzustand) vorliegend durch einen Normdruck von 1013,25 mbar und eine Normtemperatur von 293,15 K gegeben sind.

Um die Zellkulturen einer gleichmäßigen Atmosphäre aussetzen zu können, ohne dass die Gefahr von Kondensation oder Austrocknen der Zellkulturen besteht, kann die wenigstens eine Probenaufnahme zum Bereithalten der wenigstens einen Zellkultur während des Begasens in einer temperierten Expositionskammer vorgesehen sein. Wenn die Temperaturregelung zum Temperieren der Expositionskammer auf eine Temperatur zwischen 34°C und 38°C, vorzugsweise zwischen 35°C und 37°C, insbesondere von wenigstens im Wesentlichen 36°C, vorgesehen ist, kann die Temperatur beispielsweise der menschlichen Lunge simuliert werden. Außerdem kann ein Austrocknen bei hoher Zellaktivität vermieden werden.

Alternativ oder zusätzlich kann zwischen dem wenigstens einen Mischer und der wenigstens einen Expositionskammer eine Temperiereinrichtung zum Temperieren, insbesondere zum Kühlen, des Testgases auf eine Temperatur zwischen 36°C und 40°C, vorzugsweise zwischen 37°C und 39°C, insbesondere von wenigstens im Wesentlichen 38°C, vorgesehen sein. So kann das Testgas bei einer Temperatur oberhalb des Taupunkts zugeführt werden. Es besteht dann nämlich keine Gefahr einer unerwünschten Kondensation. Gleichwohl kann das Testgas mit einer sehr hohen relativen Feuchtigkeit in die Expositionskammer geleitet werden, so dass es nicht zu einem Austrocknen der Zellkulturen kommt.

Dazu sollte die relative Feuchtigkeit bei Kontakt mit den Zellkulturen ganz grundsätzlich in bevorzugter Weise zwischen 95% und 100% betragen, ohne dass es aber zur Kondensation kommt. Die Obergrenze wäre daher weiter bevorzugt 99,5% relative Feuchte. Günstig haben sich für Lungenepithelzellen relative Feuchtigkeiten zwischen 97% und 99% erwiesen. Als Zielgröße kann daher 98% relative Feuchtigkeit verwendet werden.

Die wenigstens eine Probenaufnahme zum Bereithalten der wenigstens einen Zellkultur kann zur besseren Temperierung während des Begasens in einer von dem Testgas durchströmten Zellbox vorgesehen sein, die sich innerhalb der temperierten Expositionskammer befindet. So kann die Zellbox hermetisch von einem Temperierungsbereich der Expositionskammer getrennt werden, um eine Vermischung zwischen dem Testgas und der Atmosphäre der Expositionskammer auszuschließen. Zudem kann wenigstens ein Temperatursensor zum Erfassen der Temperatur des Testgases in der Zellbox vorgesehen sein, so dass die Temperatur des Testgases in der Zellbox beim Kontakt mit den Zellkulturen ganz genau eingestellt werden kann bzw. Abweichungen vom Sollzustand erkannt und detektiert werden können.

Es kann alternativ oder zusätzlich auch eine Taupunkttemperaturmessung erfolgen, um den Taupunkt in der Zellbox zu erfassen und den Taupunkt letztlich regeln zu können. Dies kann beispielsweise über eine Variation des Drucks des wenigstens einen Wasserbehälters erfolgen.

Um eine zuverlässige Langzeitmessung sicherstellen zu können, kann eine Regelung zum Einstellen der Temperatur des Testgases in der Zellbox zwischen 35°C und 39°C, vorzugsweise zwischen 36°C und 38°C, insbesondere von 37°C (± 0,5°C), vorgesehen sein. Insbesondere kann der Sollwert der Taupunkttemperatur zwischen 34°C und 39°C, vorzugsweise zwischen 36°C und 37°C, insbesondere 36,5°C, betragen.

Für eine gleichmäßige Strömung ist es bevorzugt, wenn die wenigstens eine Zellbox eintrittsseitig und/oder austrittsseitig einen sich erweiternden Diffusor und/oder einen sich verjüngenden Diffusor aufweist. So erweitert sich der Strömungsquerschnitt langsam beim Eintritt in die Zellbox und verjüngt sich langsam beim Austritt aus der Zellbox, ohne dass es zu Totzonen oder besonderen Verwirbelungen käme. Zu demselben Zweck kann die wenigstens eine Zellbox oberhalb der wenigstens einen Probenaufnahme wenigstens ein von dem Testgas überströmtes Gitternetz und/oder Lochblech aufweisen. Dort ist dann eine tendenziell konstante Konzentration an Testkomponenten im Testgas, so dass sich zudem eine treibende Konzentrationsdifferenz für die Testkonzentration von dort zu den Zellkonturen ausbildet. Dabei ist es dann nicht bedeutsam, ob eine Probe der Zellkultur eher am Eintritt oder am Austritt der Zellbox vorgesehen ist. Mithin können in einer Zellbox auch problemlos sehr viele Pobenaufnahmen neben und/oder hintereinander in Strömungsrichtung des Testgases angeordnet sein. Die Eintrittsströmung des Testgases in die Zellbox wird gegebenenfalls vergleichmäßigt, wenn wenigstens ein Gitternetz und/oder Lochblech vorgesehen ist, das von dem Testgas durchströmt wird.

Zum exakten Analysieren der Gaszusammensetzung des die Expositionskammer bzw. die Zellbox verlassenden Gases kann wenigstens eine temperierte Probennahmeleitung vorgesehen sein. Diese kann dann mit einem Analysegerät verbunden sind, die die Gaszusammensetzung ermittelt. Alternativ oder zusätzlich kann eine Kondensationsleitung zum Kondensieren dampfförmiger Anteile des Gases in einem Kondensator vorgesehen sein. So wird die Testkomponente nicht an die Umgebung abgegeben und sicher zurückgewonnen. Damit die Kondensation nicht vorzeitig erfolgt und Leitungen zusetzt, kann die Kondensationsleitung temperiert sein.

Bei einer ersten besonders bevorzugten Ausgestaltung des Verfahrens wird der Druck im wenigstens einen Wasserbehälter und/oder Standardbehälter, vorzugsweise über wenigstens einen Druckgasanschluss und/oder wenigstens ein Reduzierventil, geregelt. Dies erlaubt eine exakte und konstante Abgabe von Wasser und/oder Standard um ein Testgas zu erzeugen, das exakt die gewünschte Zusammensetzung aufweist und diese auch beibehält. Dies erfolgt sehr präzise und effizient, wenn der Druck im Wasserbehälter und/oder im Standardbehälter konstant auf einen Wert zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, eingestellt, insbesondere, geregelt wird.

Um in der weiteren Konditionierung und Zuführung des Testgases zur Probenaufnahme bzw. zur Expositionskammer eine Kondensation sicher ausschließen zu können, kann die Temperatur des verdampften Wassers und/oder des verdampften Standards im Verdampfer auf eine Temperatur zwischen 100°C und 300°C, vorzugsweise zwischen 120°C und 200°C, insbesondere zwischen 130°C und 170°C, eingestellt, insbesondere, geregelt werden. Alternativ oder zusätzlich kann der Druck des verdampften Wassers vor der Wasserdampfkapillare und/oder der Druck des verdampften Standards vor der Dampfkapillare auf einen Druck zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, eingestellt, insbesondere, geregelt werden. Dann kann durch die wenigstens eine Wasserdampfkapillare und/oder die Dampfkapillare trotz des Verdampfens eine sehr konstante Strömung bereitgestellt werden. Die entsprechende Kapillare hat hier eine Pufferwirkung oder Ausgleichswirkung hinsichtlich des Massenstroms nach der entsprechenden Kapillare.

Dem Verdampfer des Wassers, dem Verdampfer des Standards, der Wasserdampfkapillare und/oder der Dampfkapillare kann wenigstens ein Mischer, insbesondere ein statischer Mischer, nachgeschaltet sein. So wird wenigstens das verdampfte Wasser, der verdampfte Standard und/oder das Trägergas gemischt. Alle zusammen bilden dann das Testgas, wobei auch nur zwei der genannten Massenströme im Mischer gemischt bzw. die Massenströme in mehr als einem Mischer, bedarfsweise nacheinander, gemischt werden können. Das gemeinsame Mischen in einem einzigen Mischer ist aber apparativ besonders einfach und führt dennoch, insbesondere bei der Verwendung eines statischen Mischers, zu einem gleichmäßigen Testgas.

In der wenigstens einen Konditioniereinrichtung kann im Übrigen ein vorgegebener Massenstrom an Luft und/oder an Kohlendioxid (CO2) zugeführt, insbesondere miteinander gemischt, werden. Dies dient der Bereitstellung eines geeigneten Trägergases als Teil des Testgases. Besonders zweckmäßig ist es in diesem Zusammenhang, wenn das Kohlendioxid mit einem bestimmten Massenstrom zwischen 5 Nl/h und 100 Nl/h, vorzugsweise zwischen 10 Nl/h und 50 Nl/h, insbesondere zwischen15 Nl/h und 30 Nl/h, und/oder die Luft mit einem bestimmten Massenstrom zwischen 100 Nl/h und 1200 Nl/h, vorzugsweise zwischen 200 Nl/h und 800 Nl/h, insbesondere zwischen 300 Nl/h und 500 Nl/h, zugeführt wird. Der Zuführung schließt sich dabei bevorzugt ein Vermischen in einem Mischer an, der einfach und effektiv als statischer Mischer ausgebildet sein kann.

Für die genaue Regelung des wenigstens einen Massenstroms bietet sich insbesondere ein Massendurchflussregler (Mass Flow Controller) an. Dem wenigstens einen Massendurchflussmesser kann eine Verteileinrichtung zum gleichmäßigen Aufteilen des Trägergases zum Begasen von unterschiedlichen Zellkulturen mit unterschiedlichen Testgaskonzentrationen nachgeschaltet sein, so dass nicht jeder einzelne Trägergasstrom über separate Massendurchflussmesser eingestellt werden muss. Hierzu bieten sich insbesondere kritische Düsen an, die jeweils eine sehr kleine Öffnung aufweisen. Die Öffnung ist bei allen kritischen Düsen gleich groß, so dass der Durchfluss über die kritischen Düsen in genau gleiche Anteile aufgeteilt werden kann.

Aussagekräftige Messung lassen sich ohne ein Austrocknen der Zellkulturen erreichen, wenn die wenigstens eine Probenaufnahme während des Begasens der entsprechenden Zellkultur in einer, vorzugsweise auf eine bestimmte Temperatur zwischen 34°C und 38°C, vorzugsweise zwischen 35°C und 37°C, insbesondere von wenigstens im Wesentlichen 36°C, temperierten Expositionskammer bereitgehalten wird.

Alternativ oder zusätzlich kann das Testgas unmittelbar vor dem Führen zu einer in der Expositionskammer angeordneten Zellbox auf eine bestimmte Temperatur zwischen 36°C und 40°C, vorzugsweise zwischen 37°C und 39°C, insbesondere von wenigstens im Wesentlichen 38°C, temperiert, insbesondere gekühlt werden. So wird das Testgas erst unmittelbar vor dem Eintritt in die in der Expositionskammer angeordnete Zellbox annähernd auf die Zieltemperatur gebracht, so dass ein vorzeitiges Kondensieren sicher verhindert werden kann.

Nachfolgend wird die Erfindung anhand einer lediglich ein Ausführungsbeispiel darstellenden Zeichnung näher erläutert. In der Zeichnung zeigt
- Fig. 1: eine Expositionsanlage mit einer einzigen Expositionskammer in einer schematischen Ansicht und
- Fig. 2A-B: die Zellbox der Expositionskammer aus Fig. 1 in einer Seitenansicht und einer Draufsicht.

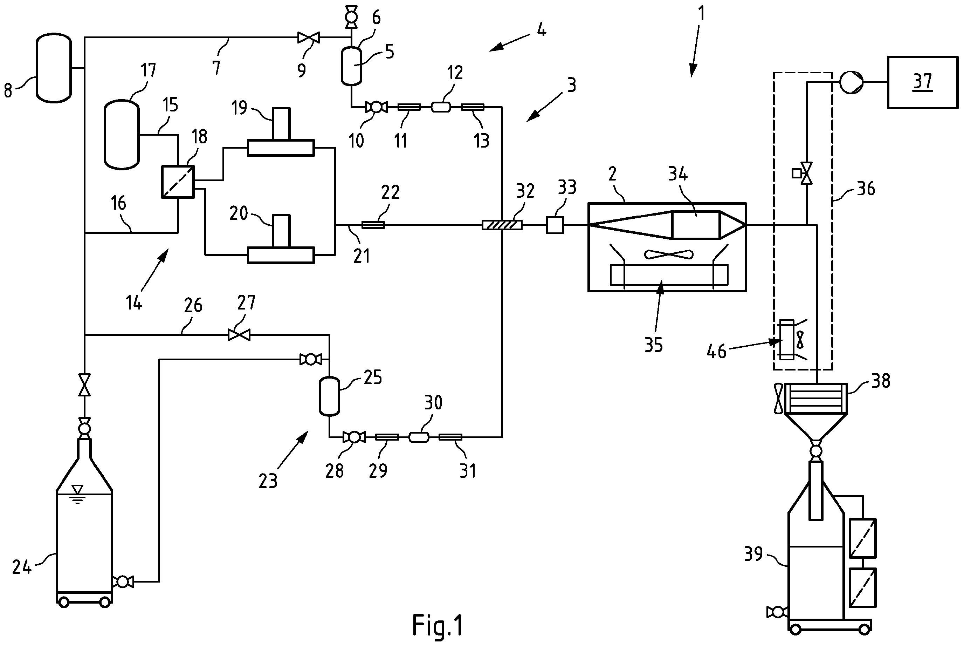

In der Fig. 1 ist eine Expositionsanalage 1 mit einer Expositionskammer 2 dargestellt, in der Zellkulturen mit einem Testgas einer bestimmten, vorgegebenen Testgaszusammensetzung begast werden können. Die Zusammensetzung des Testgases wird dabei in der Konditioniereinrichtung 3 eingestellt und geregelt, so dass die Testgaszusammensetzung auch über sehr lange Zeiträume von vielen Tagen keinen nennenswerten Schwankungen unterliegt.

Die Konditionierungseinrichtung 3 umfasst eine Testkomponentenzugabe 4, über die die gewünschte Menge einer Testkomponente zu einem Testgas hinzugefügt wird. Die Testkomponente liegt dabei in Form eines sogenannten Standards 5, beispielsweise in Reinform oder in einer bestimmten Konzentration in einer Lösung oder absorbiert in einem Absorbens vor. In dem Standard 5 ist die Konzentration der Testkomponente sehr genau bekannt und sehr genau auf einen bestimmten Wert eingestellt. Zudem liegt der Standard 5 in flüssiger Form vor, was dessen Handhabung stark vereinfacht. Der Standard 5 wird in einem Standardbehälter 6 bereitgehalten, der über einen Gasanschluss 7 an eine Gasversorgung 8 angeschlossen ist. Die Gasversorgung 8 kann dabei der Einfachheit halber durch einen Druckgasbehälter gebildet werden. Zudem kann es sich bei dem Gas der Gasversorgung 8 der Einfachheit halber um Druckluft handeln. Über ein dem Gasanschluss 8 zugeordnetes, einstellbares und/oder regelbares Reduzierventil 9, kann der Druck, mit dem der Standardbehälter 6 über den Gasanschluss 7 beaufschlagt wird, eingestellt, insbesondere geregelt, werden. Der Druck beträgt dabei vorliegend zwischen 1,3 bar und 1,5 bar. Die Temperatur des Standardbehälters 6 entspricht zudem bedarfsweise Raumtemperatur.

Über ein Absperrorgan 10 kann der Standard nun mit dem Druck des Standardbehälters 6 in eine Flüssigkeitskapillare 11 geleitet werden, die dafür sorgt, dass ein direkt mit dem Druck des Standardbehälters 6 gekoppelter und daher sehr exakt einzustellender Massenstrom an Standard 5 in einen der Flüssigkeitskapillare 11 nachgeschalteten Verdampfer 12 geleitet wird. Dort herrschen eine Temperatur von etwa 150°C und ein Druck von 1,3 bar bis 1,5 bar, so dass der Standard 5 im Verdampfer 12 vollständig verdampft wird. Dem Verdampfer 12 nachgeschaltet ist eine Dampfkapillare 13, die für eine Pufferung des Druck und des Massenstroms nach dem Verdampfer 12 sorgt. Die Flüssigkeitskapillare 11 weist dabei einen geringeren Durchmesser und eine größere Länge als die Dampfkapillare 13 auf. Dies verbessert nicht nur die Dosiergenauigkeit, sondern trägt auch den fluiddynamischen Unterschieden von flüssigen und gasförmigen Strömungen Rechnung. Der Massenstrom des Standards 5 kann beispielsweise zwischen 1 g/h und 50 g/h betragen, wobei jedoch Massenströme zwischen 2 g/h und 25 g/h, insbesondere zwischen 3 g/h und 10 g/h weiter bevorzugt sind. Vorliegend beträgt der Massenstrom des Standards etwa 5 g/h. Die Flüssigkeitskapillare 11 und die Dampfkapillare 13 können beispielsweise einen Durchmesser zwischen 0,1 mm und 0,8 mm und eine Länge bis zu 5 cm aufweisen. Dabei beträgt der Durchmesser der Flüssigkeitskapillare 11 vorzugsweise etwa 0,1 mm, während der Durchmesser der Dampfkapillare 13 vorzugsweise etwa 0,8 mm beträgt.

Die separate Trägergaszuführung 14 weist vorliegend zwei Gasanschlüsse 15,16 auf, wobei grundsätzlich die Anzahl der Gasanschlüsse variieren kann. In einem besonders einfachen Fall kann auch ein Gasanschluss ausreichen, etwa wenn der Gasanschluss bereits das Trägergas in der gewünschten Zusammensetzung bereitstellt oder ein reines Gas als Trägergas verwendet werden soll. Vorliegend wird über die Trägergaszuführung 14 ein Trägergas umfassend Luft und Kohlendioxid bereitgestellt. Daher sind die Gasanschlüsse auch als ein Druckluftanschluss und als ein Kohlendioxidanschluss ausgebildet. Auch hier werden die Luft und das Kohlendioxid der Einfachheit halber wieder über Druckgasbehälter 8,17 enthaltend einerseits Luft und andererseits Kohlendioxid bereitgestellt. Die Luft und das Kohlendioxid werden nach dem Abziehen über die jeweiligen Gasanschlüsse 15,16 durch jeweils zwei Hochtemperatur-Sterilfilter 18 gereinigt. Die Hochtemperatur-Sterilfilter 18 werden auf 180°C bis 200°C aufgeheizt, um den Hochtemperatur-Sterilfilter 18 steril zu halten, wobei das Gas aufgrund der sehr kurzen Verweilzeit im Hochtemperatur-Sterilfilter 18 weit weniger aufgeheizt wird. Sodann werden die Luft und das Kohlendioxid jeweils einem Massendurchflussregler 19,20 zugeführt, über den der jeweilige Massenstrom der Luft und des Kohlendioxids geregelt wird. Vorliegend beträgt der Massenstrom etwa 20 Nl/h (Normliter pro Stunde), während der Massenstrom der Luft etwa 380 Nl/h beträgt. Die Gasströme werden nach den Massendurchflussreglern 19,20 in einer Sammelleitung 21 zusammengeführt. Bei der dargestellten und insoweit bevorzugten Expositionsanalage ist den Massendurchflussreglern 19,20 eine Verteileinrichtung 22 zum gleichmäßigen Aufteilen des Trägergases zum Begasen von unterschiedlichen Zellkulturen mit unterschiedlichen Testgaskonzentrationen nachgeschaltet, was jedoch keineswegs zwingend ist. Das Trägergas der eingestellten Konzentrationen kann über die Verteileinrichtung 22 gleichmäßig aufgeteilt werden, ohne dass dazu weitere Massendurchflussmesser erforderlich wären. Die einzelnen Trägergasströme können dann mit unterschiedlichen Konzentrationen der Testkomponente versetzt werden, um unterschiedliche Zellkulturen, bedarfsweise der gleichen Art, parallel mit unterschiedlichen Testgaskonzentrationen zu begasen. Dann muss nicht jeder einzelne Trägergasstrom über separate Massendurchflussmesser eingestellt werden. Bevorzugt weist die Verteileinrichtung 22 eine Anzahl parallel geschalteter kritischer Düsen auf, die der Anzahl der einzelnen Trägergasströme entspricht. Die kritischen Düsen weisen jeweils eine sehr kleine Öffnung auf, wobei die Öffnungen bei allen kritischen Düsen gleich groß sind, so dass der Durchfluss problemlos über die kritischen Düsen in genau gleiche Anteile aufgeteilt werden kann. Es kann also beispielsweise eine Expositionsanlage bereitgestellt werden, die mehrere Zellboxen 2 zum Begasen von Zellkulturen mit unterschiedlichen Testgaskonzentrationen sowie dementsprechend mehrere Testkomponentenzugaben 4 und Testgasbefeuchtungen 23 umfasst. Dagegen kann dann nur eine Trägergaszuführung 14 mit einer Verteileinrichtung 22 vorgesehen sein, die Trägergasströme für jede der Zellboxen 2 bereitstellt.

Über eine Testgasbefeuchtung 23 wird der gewünschte Feuchtegehalt des Testgases eingestellt. Das Wasser zum Befeuchten des Trägergases wird im vorliegenden Fall über einen Vorlagebehälter 24 bereitgestellt, aus dem sich dann der Wasserbehälter 25 speist. Es könnte aber anstelle des Vorlagebehälters 25 auch lediglich ein Wasseranschluss vorgesehen sein. Der Wasserbehälter 25 wird über einen Druckgasanschluss 26, bei dem es sich vorliegend um einen Druckluftanschluss handelt, mit Druck beaufschlagt, der über ein Reduzierventil 27 eingestellt und/oder geregelt werden kann. Der Druck des Wasserbehälters 25 beträgt vorliegend zwischen 1,3 bar und 1,5 bar und drückt das Wasser über ein Absperrorgan 28 durch eine Wasserkapillare 29, die einen solchen Strömungswiderstand erzeugt, dass über den Druck im Wasserbehälter 25 sehr exakt der gewünschte Massenstrom Wasser zum Befeuchten des Trägergases eingestellt werden kann. Der entsprechende Massenstrom an Wasser kann beispielsweise zwischen 4 g/h und 80 g/h, vorzugsweise zwischen 8 g/h und 40 g/h, insbesondere zwischen 12 g/h und 25 g/h betragen. Vorliegend beträgt der Massenstrom an Wasser etwa 16 g/h, der nach der Wasserkapillare 29 in einen Verdampfer 30 gelangt, in dem eine Temperatur vor etwa 150°C und ein Druck zwischen 1,3 bar und 1,5 bar herrscht. Mithin wird im Verdampfer 30 das Wasser vollständig verdampft, bevor es durch eine Dampfkapillare 31 zum Puffern des Drucks und des Massenstroms geleitet wird. Die Wasserkapillare 29 weist dabei einen geringeren Durchmesser und eine größere Länge als die Wasserdampfkapillare 31 auf. Die Wasserkapillare 29 und die Wasserdampfkapillare 31 können beispielsweise einen Durchmesser zwischen 0,1 mm und 0,8 mm und eine Länge bis zu 5 cm aufweisen. Dabei beträgt der Durchmesser der Wasserkapillare 29 vorzugsweise etwa 0,1 mm, während der Durchmesser der Wasserdampfkapillare 31 vorzugsweise etwa 0,8 mm beträgt.

Der verdampfte Standard 5, das Trägergas und der Wasserdampf werden separat einem Mischer 32 zugeführt, bei dem es sich um einen statischen Mischer handelt, und in dem ein homogenes Testgas mit einer homogenen Testgaszusammensetzung erzeugt wird. Die Expositionsanlage 1 ist jedoch nicht auf die Verwendung eines einzigen Mischers 32 zu diesem Zweck beschränkt. Zwischen dem Mischer 32 und der Expositionskammer 2, vorzugsweise unmittelbar vor der Expositionskammer 2 erfolgt eine Testgastemperierung zur Einstellung der Temperatur des Testgases. Dazu wird insbesondere eine Temperiereinrichtung 33, etwa in Form eines Heizschlauchs, vorgesehen, die das Testgas auf eine Temperatur kurz oberhalb der Taupunkttemperatur temperiert. Vorliegend wird das Testgas unmittelbar vor der Expositionskammer 2 auf etwa 38°C temperiert. Das Testgas wird dann in eine Zellbox 34 eingeleitet, die sich in der temperierten Expositionskammer 2 befindet. Die Expositionskammer 2 ist vorliegend auf eine Temperatur von etwa 36°C temperiert. Hierzu ist in der dargestellten und insoweit bevorzugten Expositionskammer 2 eine Heizeinrichtung 35 mit Ventilator zur Vergleichmäßigung der Temperatur in der Expositionskammer 2 vorgesehen.

Infolge der Temperatur der Expositionskammer 2 kühlt sich das Testgas in der Expositionskammer 2 weiter ab, und zwar derart, dass die Temperatur des Testgases im Bereich der Zellkulturen etwa 37°C beträgt und somit eine relative Feuchtigkeit von etwa 98% aufweist. So kann ein Austrocknen der Zellkulturen während der Testmessungen sicher verhindert werden, ohne dass es gleichzeitig zu einer vorzeitigen Kondensation kommt. Für die Regelung der Temperatur des Testgases in der Zellbox im Bereich der Zellkulturen, sind in der Zellbox 34 zwei Temperaturmessstellen vorgesehen, eine im Testgas vor dem Erreichen der Zellkulturen und eine im Testgas nach dem Überströmen der Zellkulturen. Dach dem Überströmen der Zellkulturen kann zusätzlich oder alternativ zu der Temperaturmessung noch eine Taupunktsmessung erfolgen. Vorliegend ist der Sollwert der Taupunkttemperatur an dieser Stelle 36,5°C. Eine Regelung sorgt dafür, dass der Druck im Wasserbehälter 25 angehoben wird, sofern die Taupunkttemperatur die Solltemperatur unterschreitet, und dass der Druck im Wasserbehälter 25 gesenkt wird, wenn die Taupunkttemperatur die Solltemperatur überschreitet.

Anschließend verlässt das Testgas die Zellbox 34 und die Expositionskammer 2 und gelangt in eine beheizte Kammer 36, deren Temperatur vorzugsweise deutlich oberhalb der Taupunkttemperatur des Testgases liegt, um vorzeitige Kondensation zu vermeiden. Aus dieser Kammer 36 kann benutztes Testgas an ein Analysegerät 37 abgegeben werden, um die Zusammensetzung des benutzten Testgases zu ermitteln. Nicht zu Analysezwecken abgezogenes benutztes Testgas wird einem Kondensator 38 zugeführt, in dem die kondensierbaren Anteile des benutzten Testgases, insbesondere die Feuchtigkeit und die Testkomponente, kondensiert und damit in einem Sammelbehälter 39 abgeschieden werden. Auch in der beheizten Kammer 36 kann eine Heizeinrichtung 46 ggf. mit Ventilator zur Vergleichmäßigung der Temperatur in der beheizten Kammer 36 vorgesehen sein.

In vielen Fällen wird es bevorzugt sein, Zellkulturen zeitgleich mit Testgas unterschiedlicher Zusammensetzung zu begasen, etwa um den Einfluss unterschiedlicher Konzentrationen einer Testkomponente zu ermitteln. Dann kann die dargestellte Konditionierungseinrichtung 3 wenigstens in Teilen mehrfach vorgesehen und mit jeweils einer separaten Expositionskammer 2 verbunden sein. So kann die Konditionierung unterschiedlicher Testgase sehr genau und wenigstens im Wesentlichen unabhängig von der Konditionierung anderer Testgase erfolgen. Besonders bevorzugt ist dabei die Verwendung von 4, 6 oder 8 unterschiedlichen Expositionskammern 2, die mit Testgas unterschiedlicher Konzentration an Testkomponenten betrieben werden können, und zwar in einer einzigen Expositionsanlage.

Um den anlagentechnischen Aufwand jedoch gering zu halten, können bedarfsweise alle Konditionierungseinrichtungen an einen gemeinsamen Gasanschluss, insbesondere Druckluftanschluss und/oder Kohlendioxidanschluss angeschlossen sein. Zudem kann alternativ oder zusätzlich ein gemeinsamer Vorlagebehälter für Wasser genutzt werden, um von diesem aus die Wasserbehälter mit Wasser zu füllen bzw. zu versorgen. In einigen Fällen kann es zur Verringerung der Anlagenkosten ausreichen, wenn die Trägergaszuführung lediglich einfach vorgesehen wird. Dies gilt jedenfalls solange, wie das bedarfsweise aus mehreren Gasen durch Mischen in der Trägergaszuführung hergestellte Trägergas exakt auf die einzelnen Expositionskammern bzw. auf die Mischer zum Vermischen des Trägergases mit Wasserdampf und verdampftem Standard aufgeteilt werden kann. Es muss nämlich sichergestellt sein, dass in jedem separaten Mischer oder in jeder separaten Gruppe von Mischern jeweils ein Testgas mit einer anderen, vorgegebenen Testgaszusammensetzung erzeugt wird. Dies kann mit entsprechendem regelungstechnischen und apparativen Aufwand bedarfsweise mittels einer einzigen Testgaszuführung erreicht werden. Eine einzige Testkomponentenzugabe und eine einzige Testgasbefeuchtung wäre dagegen eher nicht ausreichend, um zeitgleich Testgase mit verschiedenen Testgaszusammensetzungen zu erzeugen.

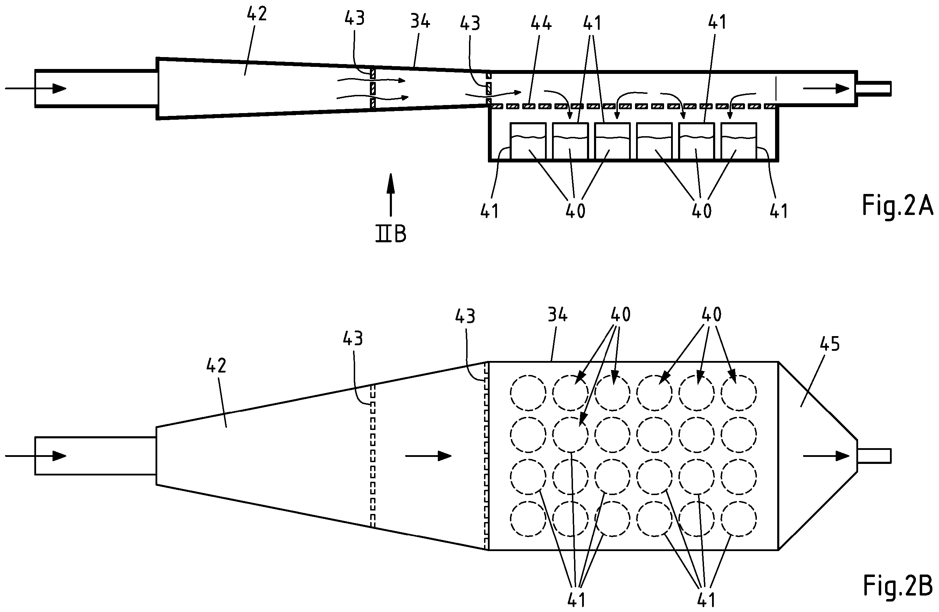

Eine strömungstechnisch optimierte Zellbox 34 zur Aufnahme der Zellkulturen 40 ist in den Fig. 2A-B dargestellt. Die Zellbox 34 weist eine Mehrzahl von nacheinander und nebeneinander angeordneten Probenaufnahmen 41 auf. Grundsätzlich wäre hier auch eine einzige Probenaufnahme 41 ausreichend. Reproduzierbarere Ergebnisse werden jedoch bei Verwendung mehrerer Probenaufnahmen 41 in einer Zellbox 34 erhalten. Die Probenaufnahmen 41 sind nach oben geöffnet, so dass die Probenaufnahmen 41 und damit die Zellkulturen 40 überströmendes Testgas mit den Zellkulturen 40 in den Probenaufnahmen 41 in Kontakt gelangen kann, ohne die Zellkuturen 40 mechanisch zu schädigen oder unter Stress zu setzen. Am Eintritt der Zellbox 34 ist ein Diffusor 42 mit einem sich stetig erweiternden Strömungsquerschnitt vorgesehen. In dem Diffusor 42 sind zudem noch Gitternetze 43 und/oder Lochbleche eingebaut, die sich quer zum gesamten Strömungsquerschnitt erstrecken und so das gesamte Testgas zum Hindurchströmen zwingen. Die so vergleichmäßigte Strömung des Testgases strömt nunmehr über ein weiteres Gitternetz 44 und/oder Lochblech, das oberhalb der Probenaufnahmen 41 angeordnet ist. So kann eine definiert und gemäßigte Strömung an Testgas zu den Probenaufnahmen 41 geleitet und nach Kontakt mit den Zellkulturen 40 wieder abgeleitet werden. Die Atmosphäre über den Zellkulturen 40 bleibt so möglichst konstant und entspricht zudem möglichst der Testgaszusammensetzung oberhalb des Gitternetzes 44 und/oder Lochblechs. Das benutzte Testgas strömt sodann über einen kürzeren Diffusor 45 am Austritt der Zellbox 34 wieder aus der Zellbox 34 aus.

### Bezugszeichenliste

- 1: Expositionsanalage
- 2: Expositionskammer
- 3: Konditioniereinrichtung
- 4: Testkomponentenzugabe
- 5: Standard
- 6: Standardbehälter
- 7: Gasanschluss
- 8: Gasversorgung
- 9: Reduzierventil
- 10: Absperrorgan
- 11: Flüssigkeitskapillare
- 12: Verdampfer
- 13: Dampfkapillare
- 14: Trägergaszuführung
- 15,16: Gasanschluss
- 17: Druckgasbehälter
- 18: Hochtemperatur-Sterilfilter
- 19,20: Massendurchflussregler
- 21: Sammelleitung
- 22: Verteileinrichtung
- 23: Testgasbefeuchtung
- 24: Vorlagebehälter
- 25: Wasserbehälter
- 26: Druckgasanschluss
- 27: Reduzierventil
- 28: Absperrorgan
- 29: Wasserkapillare
- 30: Verdampfer
- 31: Wasserdampfkapillare
- 32: Mischer
- 33: Temperiereinrichtung
- 34: Zellbox
- 35: Heizeinrichtung
- 36: beheizte Kammer
- 37: Analysegerät
- 38: Kondensator
- 39: Sammelbehälter
- 40: Zellkulturen
- 41: Probenaufnahmen
- 42: Diffusor
- 43: Gitternetz
- 44: Gitternetz
- 45: Diffusor
- 46: Heizeinrichtung

## Patentansprüche

1. Expositionsanlage (1) zum kontinuierlichen Begasen wenigstens einer Zellkultur (40) mit wenigstens einem Testgas, mit wenigstens einer Probenaufnahme (41) zum Bereithalten der wenigstens einen Zellkultur (40) während des Begasens und mit wenigstens einer Konditioniereinrichtung (3) zur Konditionierung des wenigstens einen Testgases, wobei die Konditioniereinrichtung (3) wenigstens eine Trägergaszuführung (14), wenigstens eine Testkomponentenzugabe (4) und wenigstens eine Testgasbefeuchtung (23) aufweist und wobei die wenigstens eine Testgasbefeuchtung (23) wenigstens einen Wasserbehälter (25) aufweist,
**dadurch gekennzeichnet, dass** dem Wasserbehälter (25) wenigstens eine Druckversorgung (8) zum Beaufschlagen des zum Testgas zu dosierenden Wassers im Wasserbehälter (25) mit einem vorbestimmten Druck zugeordnet ist, dass dem Wasserbehälter (25) eine Wasserkapillare (29) zum Durchleiten des zu dosierenden und mit dem vorbestimmten Druck beaufschlagten Wassers nachgeschaltet ist und dass der Wasserkapillare (29) ein Verdampfer (30) zum Verdampfen des die Wasserkapillare (29) passierenden Wassers nachgeschaltet ist.

2. Expositionsanlage (1) zum kontinuierlichen Begasen wenigstens einer Zellkultur (40) mit wenigstens einem Testgas, vorzugsweise nach Anspruch 1, mit wenigstens einer Probenaufnahme (41) zum Bereithalten der wenigstens einen Zellkultur (40) während des Begasens und mit wenigstens einer Konditioniereinrichtung (3) zur Konditionierung des wenigstens einen Testgases, wobei die Konditioniereinrichtung (3) wenigstens eine Trägergaszuführung (14), wenigstens eine Testkomponentenzugabe (4) und wenigstens eine Testgasbefeuchtung (23) aufweist, wobei die Testkomponentenzugabe (4) wenigstens einen Standardbehälter (6) zum Bereithalten wenigstens eines die wenigstens eine zu dosierende Testkomponente aufweisenden Standards (5) aufweist und wobei der Standardbehälter (6) wenigstens eine Druckversorgung (8) zum Beaufschlagen des zu dosierenden Standards (5) im Standardbehälter (6) mit einem vorbestimmten Druck zugeordnet ist,
**dadurch gekennzeichnet, dass** dem Standardbehälter (6) eine Flüssigkeitskapillare (11) zum Durchleiten des zu dosierenden und mit dem vorbestimmten Druck beaufschlagten Standards (5) nachgeschaltet ist und dass der Flüssigkeitskapillare (11) ein Verdampfer (12) zum Verdampfen des die Flüssigkeitskapillare (11) passierenden Standards (5) nachgeschaltet ist.

3. Expositionsanlage nach Anspruch 1 oder 2,
**dadurch gekennzeichnet, dass** die Druckversorgung (8) des Wasserbehälters (25) und/oder des Standardbehälters (6) wenigstens einen Druckgasanschluss, insbesondere Druckluftanschluss, und/oder wenigstens eine Druckregelung zur Regelung des Drucks im Wasserbehälter (25) und/oder im Standardbehälter (6)aufweist.

4. Expositionsanlage nach einem der Ansprüche 1 bis 3,
**dadurch gekennzeichnet, dass** der Druck im Wasserbehälter (25) und/oder im Standardbehälter (6) zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, beträgt.

5. Expositionsanlage nach einem der Ansprüche 1 bis 4,
**dadurch gekennzeichnet, dass** dem Verdampfer (12,30) zum Verdampfen des Wassers und/oder des Standards (5) wenigstens eine Verdampferregelung zur Regelung der Temperatur des verdampften Wassers und/oder des verdampften Standards (5) zugeordnet ist und dass, vorzugsweise, die Temperatur des verdampften Wassers und/oder Standards (5) im Verdampfer (12,30) zwischen 100°C und 300°C, vorzugsweise zwischen 120°C und 200°C, insbesondere zwischen 130°C und 170°C, und/oder der Druck des verdampften Wassers vor einer Wasserdampfkapillare (31) und/oder des verdampften Standards vor einer Dampfkapillare (13) zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, beträgt.

6. Expositionsanlage nach einem der Ansprüche 1 bis 5,
**dadurch gekennzeichnet, dass** dem Verdampfer (30) des Wassers, dem Verdampfer (12) des Standards (5), der Wasserdampfkapillare (31) und/oder der Dampfkapillare (13) ein Mischer (32), insbesondere ein statischer Mischer (32), zum Vermischen des verdampften Wassers und des verdampften Standards (5) untereinander und/oder zum Vermischen des verdampften Wassers und/oder des verdampften Standards (5) mit dem Trägergas nachgeschaltet ist.

7. Expositionsanlage nach einem der Ansprüche 1 bis 6,
**dadurch gekennzeichnet, dass** die Trägergaszuführung (14) wenigstens einen Gasanschluss (15,16), wenigstens einen Luftanschluss und/oder Kohlendioxidanschluss (CO₂), umfasst und dass, vorzugsweise, dem wenigstens einen Gasanschluss (15,16) ein Massendurchflussregler (19,20) zum Regeln des Massenstroms des über den Gasanschluss (15,16) zugeführten Gases, insbesondere Trägergases, und/oder dem wenigstens einen Gasanschluss (15,16) und/oder dem wenigstens einen Massendurchflussregler (19,20) eine Verteileinrichtung (22), insbesondere mit einer Mehrzahl von kritischen Düsen, zum gleichmäßigen Aufteilen des Trägergases nachgeschaltet ist.

8. Expositionsanlage nach Anspruch 7,
**dadurch gekennzeichnet, dass** wenigstens ein Massendurchflussregler (19) zum Regeln des Massenstroms des über den Gasanschluss (15) zugeführten Kohlendioxids, zwischen 5 Nl/h und 100 Nl/h, vorzugsweise zwischen 10 Nl/h und 50 Nl/h, insbesondere zwischen 15 Nl/h und 30 Nl/h, ausgebildet ist und/oder wenigstens ein Massendurchflussregler (20) zum Regeln des Massenstroms der über den Gasanschluss (16) zugeführten Luft, zwischen 100 Nl/h und 1200 Nl/h, vorzugsweise zwischen 200 Nl/h und 800 Nl/h, insbesondere zwischen 300 Nl/h und 500 Nl/h, ausgebildet ist.

9. Expositionsanlage nach einem der Ansprüche 1 bis 8,
**dadurch gekennzeichnet, dass** die wenigstens eine Probenaufnahme (41) zum Bereithalten der wenigstens einen Zellkultur (40) während des Begasens in einer temperierten Expositionskammer (2) vorgesehen ist und dass, vorzugsweise, die Temperaturregelung zum Temperieren der Expositionskammer (2) auf eine Temperatur zwischen 34°C und 38°C, vorzugsweise zwischen 35°C und 37°C, insbesondere von wenigstens im Wesentlichen 36°C, vorgesehen ist.

10. Expositionsanlage nach Anspruch 9,
**dadurch gekennzeichnet, dass** zwischen dem wenigstens einen Mischer (32) und der wenigstens einen Expositionskammer (2) eine Temperiereinrichtung (33) zum Temperieren des Testgases auf eine Temperatur zwischen 36°C und 40°C, vorzugsweise zwischen 37°C und 39°C, insbesondere von wenigstens im Wesentlichen 38°C, vorgesehen ist.

11. Expositionsanlage nach Anspruch 9 oder 10,
**dadurch gekennzeichnet, dass** die wenigstens eine Probenaufnahme (41) zum Bereithalten der wenigstens einen Zellkultur (40) während des Begasens in einer von dem Testgas durchströmten Zellbox (34) in der temperierten Expositionskammer (2) vorgesehen ist und dass, vorzusgweise, wenigstens ein Temperatursensor zum Erfassen der Temperatur des Testgases in der Zellbox (34) vorgesehen ist.

12. Expositionsanlage nach Anspruch 11,
**dadurch gekennzeichnet, dass** eine Regelung zum Einstellen der Temperatur des Testgases in der Zellbox (34) zwischen 35°C und 39°C, vorzugsweise zwischen 36°C und 38°C, insbesondere von 37°C (± 0,5°C), vorgesehen ist.

13. Expositionsanlage nach Anspruch 11 oder 12,
**dadurch gekennzeichnet, dass** die wenigstens eine Zellbox (34) eintrittsseitig und/oder austrittsseitig einen sich erweiternden Diffusor (42) und/oder einen sich verjüngenden Diffusor (45) aufweist und/oder dass die wenigstens eine Zellbox (34) oberhalb der wenigstens einen Probenaufnahme (41) wenigstens ein von dem Testgas überströmtes Gitternetz (44) und/oder Lochblech vorgesehen ist und/oder dass eintrittsseitig in der wenigstens einen Zellbox (34) wenigstens ein von dem Testgas durchströmtes Gitternetz (43) und/oder Lochblech vorgesehen ist.

14. Expositionsanlage nach einem der Ansprüche 9 bis 13,
**dadurch gekennzeichnet, dass** der wenigstens einen Expositionskammer (2) eine temperierte Probennahmeleitung zum Analysieren der Gaszusammensetzung und/oder eine, vorzugsweise temperierte, Kondensationsleitung zum Kondensieren dampfförmiger Anteile des Gases in einem Kondensator (38) nachgeschaltet ist.

15. Verfahren zum Betreiben einer Expositionsanlage (1) zum kontinuierlichen Begasen wenigstens einer Zellkultur (40) in wenigstens einer Probenaufnahme (41) mit wenigstens einem Testgas, insbesondere nach einem der Ansprüche 1 bis 14,
- bei dem in wenigstens einer Konditioniereinrichtung (3) das wenigstens eine Testgas zum Begasen der wenigstens einen Zellkultur (40) konditioniert wird,
- bei dem in der wenigstens einen Konditioniereinrichtung (3), insbesondere wenigstens einer Testgasbefeuchtung (23), zu dosierendes Wasser aus wenigstens einem durckbeaufschlagten Wasserbehälter (25) mit einem vorbestimmten Druck einer Wasserkapillare (29) zugeführt und das Wasser nach dem Verlassen der Wasserkapillare (29) verdampft wird und/oder
- bei dem in der wenigstens einen Konditioniereinrichtung (3), insbesondere wenigstens einer Testkomponentenzugabe (4), zu dosierender Standard (5) aus wenigstens einem druckbeaufschlagten Standardbehälter (6) mit einem vorbestimmten Druck einer Flüssigkeitskapillare (11) zugeführt und der Standard (5) nach dem Verlassen der Flüssigkeitskapillare (11) verdampft wird.

16. Verfahren nach Anspruch 15,
- bei dem der Druck im wenigstens einen Wasserbehälter (25) und/oder Standardbehälter (6), vorzugsweise über wenigstens einen Druckgasanschluss (8) und/oder wenigstens ein Reduzierventil (27), geregelt wird und
- bei dem, vorzugsweise, der Druck im Wasserbehälter (25) und/oder im Standardbehälter (6) konstant auf einen Wert zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, geregelt wird.

17. Verfahren nach Anspruch 15 oder 16,
- bei dem die Temperatur des verdampften Wassers und/oder des verdampften Standards (5) im Verdampfer (12,30) auf eine Temperatur zwischen 100°C und 300°C, vorzugsweise zwischen 120°C und 200°C, insbesondere zwischen 130°C und 170°C, geregelt wird und/oder
- bei dem der Druck des verdampften Wassers vor der Wasserdampfkapillare (13) und/oder der Druck des verdampften Standards (5) vor der Dampfkapillare (31) auf einen Druck zwischen 1 bar und 4 bar, vorzugsweise zwischen 1,1 bar und 3 bar, insbesondere zwischen 1,2 bar und 2 bar, weiter insbesondere zwischen 1,3 bar und 1,5 bar, geregelt wird.

18. Verfahren nach einem der Ansprüche 15 bis 17,
bei dem dem Verdampfer (30) des Wassers, dem Verdampfer (12) des Standards (5), der Wasserdampfkapillare (31) und/oder der Dampfkapillare (13) nachgeschaltet in wenigstens einem Mischer (32), insbesondere statischem Mischer (32), verdampftes Wassers, verdampfter Standard (5) und/oder Trägergas, insbesondere zum Testgas, gemischt werden.

19. Verfahren nach einem der Ansprüche 15 bis 18,
- bei dem in der wenigstens einen Konditioniereinrichtung (3) ein vorgegebener Massenstrom an Luft und/oder an Kohlendioxid (CO₂) zugeführt, insbesondere gemischt, wird und
- bei dem, vorzugsweise, Kohlendioxid mit einem bestimmten Massenstrom zwischen 5 Nl/h und 100 Nl/h, vorzugsweise zwischen 10 Nl/h und 50 Nl/h, insbesondere zwischen15 Nl/h und 30 Nl/h, und/oder Luft mit einem bestimmten Massenstrom zwischen 100 Nl/h und 1200 Nl/h, vorzugsweise zwischen 200 Nl/h und 800 Nl/h, insbesondere zwischen 300 Nl/h und 500 Nl/h, zugeführt wird.

20. Verfahren nach Anspruch 19,
- bei dem der wenigstens eine Massenstrom über wenigstens einen Massendurchflussregler (19,20) geregelt wird und
- bei dem, vorzugsweise, das Gas nach einem Massendurchflussmesser (19,20) eine Verteileinrichtung (22) durchströmt.

21. Verfahren nach einem der Ansprüche 15 bis 20,
- bei dem die wenigstens eine Probenaufnahme (41) während des Begasens der entsprechenden Zellkultur (40) in einer, vorzugsweise auf eine bestimmte Temperatur zwischen 34°C und 38°C, vorzugsweise zwischen 35°C und 37°C, insbesondere von wenigstens im Wesentlichen 36°C, temperierte Expositionskammer (2) bereitgehalten wird und/oder
- bei dem das Testgases unmittelbar vor dem Führen zu einer in der Expositionskammer (2) angeordneten Zellbox (34) auf eine bestimmte Temperatur zwischen 36°C und 40°C, vorzugsweise zwischen 37°C und 39°C, insbesondere von wenigstens im Wesentlichen 38°C, temperiert, insbesondere abgekühlt wird.

22. Verfahren nach Anspruch 21,
- bei dem wenigstens eine Temperatur des Testgases in der Zellbox (34) erfasst und, vorzugsweise geregelt, wird und
- bei dem, vorzugsweise, die wenigstens eine Temperatur des Testgases in der Zellbox (34) auf eine bestimmte Temperatur zwischen 35°C und 39°C, vorzugsweise zwischen 36°C und 38°C, insbesondere von 37°C (± 0,5°C), geregelt wird.

23. Verfahren nach Anspruch 21 oder 22,
- bei dem das Testgas in der Zellbox (34) eintrittsseitig und/oder austrittsseitig einen sich erweiternden Diffusor (42) und/oder einen sich verjüngenden Diffusor (45) passiert und/oder
- bei dem das Testgas in der Zellbox (34) teilweise wenigstens ein Gitternetz (44) und/oder Lochblech oberhalb der wenigstens einen Probenaufnahme (41) überströmt und/oder
- bei dem das Testgas in der Zellbox (34) eintrittsseitig wenigstens ein Gitternetz (43) und/oder Lochblech durchströmt.

24. Verfahren nach einem der Ansprüche 15 bis 23,
- bei dem das die wenigstens eine Expositionskammer (2) verlassende Gas, insbesondere teilweise, über eine temperierte Probennahmeleitung zu einer Analyseeinrichtung (37) zum Analysieren der Gaszusammensetzung geleitet wird und/oder
- bei dem das die wenigstens eine Expositionskammer (2) verlassende Gas, wenigstens teilweise, über eine, vorzugsweise temperierte, Kondensationsleitung zu einem Kondensator (38) zum Kondensieren dampfförmiger Anteile des Gases geleitet wird.

## Claims

1. Exposure system (1) for continuously gassing at least one cell culture (40) with at least one test gas, having at least one sample receptacle (41) for holding the at least one cell culture (40) during the gassing and having at least one conditioning apparatus (3) for conditioning the at least one test gas, wherein the conditioning apparatus (3) has at least one carrier gas supply system (14), at least one test component addition system (4) and at least one test gas humidification system (23) and wherein the at least one test gas humidification system (23) has at least one water container (25),
**characterised in that**
at least one pressure supply (8) for applying a predetermined pressure to the water to be dosed to the test gas in the water container (25) is assigned to the water container (25), **in that** a water capillary (29) for channelling the water to be dosed and to which the predetermined pressure is applied is arranged downstream of the water container (25) and **in that** an evaporator (30) for evaporating the water passing the water capillary (29) is arranged downstream of the water capillary (29).

2. Exposure system (1) for continuously gassing at least one cell culture (40) with at least one test gas, preferably according to claim 1, having at least one sample receptacle (41) for holding the at least one cell culture (40) during the gassing and having at least one conditioning apparatus (3) for conditioning the at least one test gas, wherein the conditioning apparatus (3) has at least one carrier gas supply system (14), at least one test component addition system (4) and at least one test gas humidification system (23), wherein the test component addition system (4) has at least one standard container (6) for holding at least one standard (5) having the at least one test component to be dosed and wherein the standard container (6) is assigned at least one pressure supply (8) for applying a predetermined pressure to the standard (5) to be dosed in the standard container (6), **characterised in that**
a liquid capillary (11) for channelling the standard (5) to be dosed and to which the predetermined pressure is applied is arranged downstream of the standard container (6) and **in that** an evaporator (12) for evaporating the standard (5) passing the liquid capillary (11) is arranged downstream of the liquid capillary (11).

3. Exposure system according to claim 1 or 2,
**characterised in that**
the pressure supply (8) of the water container (25) and/or of the standard container (6) has at least one compressed gas connection, in particular compressed air connection, and/or has at least one pressure regulation system for regulating the pressure in the water container (25) and/or in the standard container (6).

4. Exposure system according to any one of claims 1 to 3,
**characterised in that**
the pressure in the water container (25) and/or in the standard container (6) is between 1 bar and 4 bar, preferably between 1.1 bar and 3 bar, in particular between 1.2 bar and 2 bar, more particularly between 1.3 bar and 1.5 bar.

5. Exposure system according to any one of claims 1 to 4,
**characterised in that**
at least one evaporator regulation system for regulating the temperature of the evaporated water and/or of the evaporated standard (5) is assigned to the evaporator (12, 30) for evaporating the water and/or the standard (5) and **in that**, preferably, the temperature of the evaporated water and/or standard (5) in the evaporator (12, 30) is between 100°C and 300°C, preferably between 120°C and 200°C, in particular between 130°C and 170°C and/or the pressure of the evaporated water before a water vapour capillary (31) and/or of the evaporated standard before a vapour capillary (13) is between 1 bar and 4 bar, preferably between 1.1 bar and 3 bar, in particular between 1.2 bar and 2 bar, more particularly between 1.3 bar and 1.5 bar.

6. Exposure system according to any one of claims 1 to 5,
**characterised in that**
a mixer (32), in particular a static mixer (32), for mixing the evaporated water and the evaporated standard (5) with one another and/or for mixing the evaporated water and/or the evaporated standard (5) with the carrier gas is arranged downstream of the evaporator (30) of the water, the evaporator (12) of the standard (5), the water vapour capillary (31) and/or the vapour capillary (13).

7. Exposure system according to any one of claims 1 to 6,
**characterised in that**
the carrier gas supply system (14) comprises at least one gas connection (15, 16), at least one air connection and/or carbon dioxide connection (CO₂) and **in that**, preferably, a mass flow regulator (19, 20) for regulating the mass flow of the gas, in particular carrier gas, supplied via the gas connection (15, 16) is arranged downstream of the at least one gas connection (15, 16) and/or a distributor apparatus (22), in particular with a plurality of critical nozzles, for evenly dividing the carrier gas is arranged downstream of the at least one gas connection (15, 16) and/or the at least one mass flow regulator (19, 20).

8. Exposure system according to claim 7,
**characterised in that**
at least one mass flow regulator (19) is designed for regulating the mass flow of the carbon dioxide supplied via the gas connection (15) between 5 Nl/h and 100 Nl/h, preferably between 10 Nl/h and 50 Nl/h, in particular between 15 Nl/h and 30 Nl/h and/or at least one mass flow regulator (20) is designed for regulating the mass flow of the air supplied via the gas connection (16) between 100 Nl/h and 1200 Nl/h, preferably between 200 Nl/h and 800 Nl/h, in particular between 300 Nl/h and 500 Nl/h.

9. Exposure system according to any one of claims 1 to 8,
**characterised in that**
the at least one sample receptacle (41) is provided for holding the at least one cell culture (40) during the gassing in a temperature-regulated exposure chamber (2) and **in that**, preferably, the temperature regulation is provided for regulating the temperature of the exposure chamber (2) to a temperature of between 34°C and 38°C, preferably of between 35°C and 37°C, in particular of at least substantially 36°C.

10. Exposure system according to claim 9,
**characterised in that**
a temperature regulating apparatus (33) for regulating the temperature of the test gas to a temperature of between 36°C and 40°C, preferably of between 37°C and 39°C, in particular of at least substantially 38°C, is provided between the at least one mixer (32) and the at least one exposure chamber (2).

11. Exposure system according to claim 9 or 10,
**characterised in that**
the at least one sample receptacle (41) is provided for holding the at least one cell culture (40) during the gassing in a cell box (34) flowed through by the test gas in the temperature-regulated exposure chamber (2) and **in that**, preferably, at least one temperature sensor is provided for recording the temperature of the test gas in the cell box (34).

12. Exposure system according to claim 11,
**characterised in that**
a regulation system is provided for setting the temperature of the test gas in the cell box (34) between 35°C and 39°C, preferably between 36°C and 38°C, in particular 37°C (± 0.5°C).

13. Exposure system according to claim 11 or 12,
**characterised in that**
the at least one cell box (34) has, on the inlet side and/or outlet side, an expanding diffuser (42) and/or a narrowing diffuser (45) and/or **in that** the at least one cell box (34) above the at least one sample receptacle (41) is provided with at least one grid (44) and/or perforated plate flowed over by the test gas and/or **in that** at least one grid (43) and/or perforated plate flowed through by the test gas is provided on the inlet side in the at least one cell box (34).

14. Exposure system according to any one of claims 9 to 13,
**characterised in that**
a temperature-regulated sample taking line for analysing the gas composition and/or a, preferably temperature-regulated, condensation line for condensing vaporous portions of the gas in a condenser (38) is arranged downstream of the at least one exposure chamber (2).

15. Method for operating an exposure system (1) for continuously gassing at least one cell culture (40) in at least one sample receptacle (41) with at least one test gas, in particular according to any one of claims 1 to 14,
- in which, in at least one conditioning apparatus (3), the at least one test gas for gassing the at least one cell culture (40) is conditioned,
- in which, in the at least one conditioning apparatus (3), in particular at least one test gas humidification system (23), water to be dosed is supplied from at least one pressurised water container (25) at a predetermined pressure to a water capillary (29) and the water is evaporated after leaving the water capillary (29) and/or
- in which, in the at least one conditioning apparatus (3), in particular at least one test component addition system (4), standard (5) to be dosed is supplied from at least one pressurised standard container (6) at a predetermined pressure to a liquid capillary (11) and the standard (5) is evaporated after leaving the liquid capillary (11).

16. Method according to claim 15,
- in which the pressure in the at least one water container (25) and/or standard container (6) is regulated preferably via at least one compressed gas connection (8) and/or at least one reducing valve (27) and
- in which, preferably, the pressure in the water container (25) and/or in the standard container (6) is regulated constantly to a value of between 1 bar and 4 bar, preferably of between 1.1 bar and 3 bar, in particular of between 1.2 bar and 2 bar, more particularly of between 1.3 bar and 1.5 bar.

17. Method according to claim 15 or 16,
- in which the temperature of the evaporated water and/or of the evaporated standard (5) in the evaporator (12, 30) is regulated to a temperature of between 100°C and 300°C, preferably of between 120°C and 200°C, in particular of between 130°C and 170°C and/or
- in which the pressure of the evaporated water before the water vapour capillary (13) and/or the pressure of the evaporated standard (5) before the vapour capillary (31) is regulated to a pressure of between 1 bar and 4 bar, preferably of between 1.1 bar and 3 bar, in particular of between 1.2 bar and 2 bar, more particularly of between 1.3 bar and 1.5 bar.

18. Method according to any one of claims 15 to 17,
in which evaporated water, evaporated standard (5) and/or carrier gas are mixed, in particular to form the test gas, in at least one mixer (32), in particular a static mixer (32), arranged downstream of the evaporator (30) of the water, the evaporator (12) of the standard (5), the water vapour capillary (31) and/or the vapour capillary (13).

19. Method according to any one of claims 15 to 18,
- in which, in the at least one conditioning apparatus (3), a predefined mass flow of air and/or of carbon dioxide (CO₂) is supplied, in particular mixed, and
- in which, preferably, carbon dioxide is supplied at a determined mass flow of between 5 Nl/h and 100 Nl/h, preferably of between 10 Nl/h and 50 Nl/h, in particular of between 15 Nl/h and 30 Nl/h and/or air is supplied at a determined mass flow of between 100 Nl/h and 1200 Nl/h, preferably of between 200 Nl/h and 800 Nl/h, in particular of between 300 Nl/h and 500 Nl/h.

20. Method according to claim 19,
- in which the at least one mass flow is regulated via at least one mass flow regulator (19, 20) and
- in which, preferably, the gas flows through a distributor apparatus (22) after a mass flow regulator (19, 20).

21. Method according to any one of claims 15 to 20,
- in which the at least one sample receptacle (41) is held during the gassing of the corresponding cell culture (40) in an exposure chamber (2) which is temperature-regulated preferably to a determined temperature of between 34°C and 38°C, preferably of between 35°C and 37°C, in particular of at least substantially 36°C and/or
- in which the test gas is temperature-regulated, in particular cooled, directly before being guided to a cell box (34) arranged in the exposure chamber (2) to a determined temperature of between 36°C and 40°C, preferably of between 37°C and 39°C, in particular of at least substantially 38°C.

22. Method according to claim 21,
- in which at least one temperature of the test gas in the cell box (34) is recorded and, preferably, regulated, and
- in which, preferably, the at least one temperature of the test gas in the cell box (34) is regulated to a determined temperature of between 35°C and 39°C, preferably of between 36°C and 38°C, in particular of 37°C (± 0.5°C).

23. Method according to claim 21 or 22,
- in which the test gas in the cell box (34) passes, on the inlet side and/or outlet side, an expanding diffuser (42) and/or a narrowing diffuser (45) and/or
- in which the test gas in the cell box (34) partially flows over at least one grid (44) and/or perforated plate above the at least one sample receptacle (41) and/or
- in which the test gas in the cell box (34), on the inlet side, flows through at least one grid (43) and/or perforated plate.

24. Method according to any one of claims 15 to 23,
- in which the gas leaving the at least one exposure chamber (2) is channelled, in particular partially, via a temperature-regulated sample taking line to an analysis apparatus (37) for analysing the gas composition and/or
- in which the gas leaving the at least one exposure chamber (2) is channelled, at least partially, via a, preferably temperature-regulated, condensation line to a condenser (38) for condensing vaporous portions of the gas.

## Revendications

1. Installation d'exposition (1) pour la gazéification en continu d'au moins une culture cellulaire (40) avec au moins un gaz d'essai, avec au moins un logement d'échantillon (41) pour mettre à disposition ladite au moins une culture cellulaire (40) pendant la gazéification et avec au moins un dispositif de conditionnement (3) permettant de conditionner ledit au moins un gaz d'essai, le dispositif de conditionnement (3) présentant au moins une amenée de gaz porteur (14), au moins une addition de composant d'essai (4) et au moins une humidification de gaz d'essai (23), et ladite au moins une humidification de gaz d'essai (23) présentant au moins un réservoir d'eau (25),
**caractérisée en ce que**
au moins une alimentation en pression (8) est associée au réservoir d'eau (25) pour pressuriser l'eau à doser pour le gaz d'essai dans le réservoir d'eau (25) à une pression prédéterminée, **en ce qu'**un capillaire d'eau (29) est monté en aval du réservoir d'eau (25) pour faire passer l'eau à doser et pressurisée à la pression prédéterminée et **en ce qu'**un évaporateur (30) est monté en aval du capillaire d'eau (29) afin d'évaporer l'eau passant par le capillaire d'eau (29).

2. Installation d'exposition (1) pour la gazéification en continu d'au moins une culture cellulaire (40) avec au moins un gaz d'essai, de préférence selon la revendication 1, avec au moins un logement d'échantillon (41) pour mettre à disposition ladite au moins une culture cellulaire (40) pendant la gazéification et avec au moins un dispositif de conditionnement (3) permettant de conditionner ledit au moins un gaz d'essai, le dispositif de conditionnement (3) présentant au moins une amenée de gaz porteur (14), au moins une addition de composant d'essai (4) et au moins une humidification de gaz d'essai (23), l'addition de composant d'essai (4) présentant au moins un réservoir de standard (6) pour mettre à disposition au moins un standard (5) présentant ledit au moins un composant d'essai à doser et au moins une alimentation en pression (8) étant associée au réservoir de standard (6) pour pressuriser le standard à doser (5) dans le réservoir de standard (6) à une pression prédéterminée,
**caractérisée en ce que**
un capillaire de liquide (11) est monté en aval du réservoir de standard (6) pour le passage du standard (5) à doser et pressurisé à la pression prédéterminée, et **en ce qu'**un évaporateur (12) est monté en aval du capillaire de liquide (11) pour l'évaporation du standard (5) passant par le capillaire de liquide (11).

3. Installation d'exposition selon la revendication 1 ou 2,
**caractérisée en ce que**
l'alimentation en pression (8) du réservoir d'eau (25) et/ou du réservoir de standard (6) présente au moins un raccordement au gaz comprimé, notamment un raccordement d'air comprimé, et/ou au moins une régulation de pression pour régler la pression dans le réservoir d'eau (25) et/ou le réservoir de standard (6).

4. Installation d'exposition selon l'une des revendications 1 à 3,
**caractérisée en ce que**
la pression dans le réservoir d'eau (25) et/ou dans le réservoir de standard (6) se situe entre 1 bar et 4 bars, de préférence entre 1,1 bar et 3 bars, notamment entre 1,2 bar et 2 bars, plus particulièrement entre 1,3 bar et 1,5 bar.

5. Installation d'exposition selon l'une des revendications 1 à 4,
**caractérisée en ce que**
au moins une régulation d'évaporateur, pour la régulation de la température de l'eau évaporée et/ou du standard (5) évaporé, est associée à l'évaporateur (12,30) pour l'évaporation de l'eau et/ou du standard (5), et **en ce que**, de préférence, la température de l'eau évaporée et/ou du standard évaporé dans l'évaporateur (12, 30) est comprise entre 100 °C et 300 °C, de préférence entre 120 °C et 200 °C, notamment entre 130 °C et 170 °C, et/ou la pression de l'eau évaporée en amont d'un capillaire de vapeur d'eau (31) et/ou du standard évaporé en amont d'un capillaire de vapeur (13) est comprise entre 1 bar et 4 bars, de préférence entre 1,1 bar et 3 bars, notamment entre 1,2 bar et 2 bars, plus particulièrement entre 1,3 bar et 1,5 bar.

6. Installation d'exposition selon l'une des revendications 1 à 5,
**caractérisée en ce que**
un mélangeur (32), notamment un mélangeur (32) statique, est monté en aval de l'évaporateur (30) d'eau, de l'évaporateur (12) de standard (5), du capillaire de vapeur d'eau (31) et/ou du capillaire de vapeur (13) pour mélanger l'eau évaporée et le standard évaporé (5) entre eux et/ou pour mélanger l'eau évaporée et/ou le standard évaporé (5) avec le gaz porteur.

7. Installation d'exposition selon l'une des revendications 1 à 6,
**caractérisée en ce que**
l'amenée de gaz porteur (14) comporte au moins un raccordement au gaz (15, 16), au moins un raccordement à l'air et/ou un raccordement au dioxyde de carbone (CO₂), et **en ce que**, de préférence, un régulateur de débit massique (19, 20) est associé à ledit au moins un raccordement au gaz (15, 16) pour régler le débit massique du gaz, notamment du gaz porteur, acheminé par le raccordement au gaz (15, 16) et/ou un dispositif répartiteur (22), notamment avec une multitude de buses critiques, est monté en aval dudit au moins un raccordement au gaz (15, 16) et/ou dudit au moins un régulateur de débit massique (19, 20) pour répartir uniformément le gaz porteur.

8. Installation d'exposition selon la revendication 7,
**caractérisée en ce que**
au moins un régulateur de débit massique (19) pour régler le débit massique de dioxyde de carbone acheminé par le raccordement au gaz (15) est conçu entre 5 Nl/h et 100 Nl/h, de préférence entre 10 Nl/h et 50 Nl/h, notamment entre 15 Nl/h et 30 Nl/h, et/ou ledit au moins un régulateur de débit massique (20) pour régler le débit massique de l'air acheminé par le raccordement au gaz (16) est conçu entre 100 Nl/h et 1 200 Nl/h, de préférence entre 200 Nl/h et 800 Nl/h, notamment entre 300 Nl/h et 500 Nl/h.

9. Installation d'exposition selon l'une des revendications 1 à 8,
**caractérisée en ce que**
l'on prévoit ledit au moins un logement d'échantillon (41) pour mettre à disposition ladite au moins une culture cellulaire (40) pendant la gazéification dans une chambre d'exposition (2) conditionnée thermiquement et **en ce que**, de préférence, l'on prévoit la régulation de température pour le conditionnement thermique de la chambre d'exposition (2) à une température entre 34 °C et 38 °C, de préférence entre 35 °C et 37 °C, notamment au moins essentiellement à 36 °C.

10. Installation d'exposition selon la revendication 9,
**caractérisée en ce que**
l'on prévoit, entre ledit au moins un mélangeur (32) et ladite au moins une chambre d'exposition (2), un dispositif de conditionnement thermique (33) pour le conditionnement thermique du gaz d'essai à une température comprise entre 36 °C et 40 °C, de préférence entre 37 °C et 39 °C, notamment au moins essentiellement à 38 °C.

11. Installation d'exposition selon la revendication 9 ou10,
**caractérisée en ce que**
l'on prévoit ledit au moins un logement d'échantillon (41) pour mettre à disposition ladite au moins une culture cellulaire (40) pendant la gazéification dans une boîte cellulaire (34), traversée par le gaz d'essai, dans la chambre d'exposition (2) conditionnée thermiquement et **en ce que**, de préférence, l'on prévoit au moins un capteur de température pour détecter la température du gaz d'essai dans la boîte cellulaire (34).

12. Installation d'exposition selon la revendication 11,
**caractérisée en ce que**
l'on prévoit une régulation pour ajuster la température du gaz d'essai dans la boîte cellulaire (34) entre 35 °C et 39 °C, de préférence entre 36 °C et 38 °C, notamment à 37 °C (± 0,5 °C).

13. Installation d'exposition selon la revendication 11 ou 12,
**caractérisée en ce que**
ladite au moins une boîte cellulaire (34) présente, côté entrée et/ou côté sortie, un diffuseur (42) s'élargissant et/ou un diffuseur (45) se rétrécissant et/ou **en ce que** ladite au moins une boîte cellulaire (34) comporte, au-dessus dudit au moins un logement d'échantillon (41), au moins un treillis (44) et/ou une tôle perforée traversé(e) par le gaz d'essai et/ou **en ce que** l'on prévoit, côté entrée, dans ladite au moins une boîte cellulaire (34), au moins un treillis (43) et/ou une tôle perforée traversé(e) par le gaz d'essai.

14. Installation d'exposition selon l'une des revendications 9 à 13,
**caractérisée en ce que**
une conduite pour les prises d'échantillons conditionnée thermiquement pour analyser la composition du gaz et/ou une conduite de condensation, de préférence conditionnée thermiquement, pour condenser des parties à l'état de vapeur du gaz dans un condenseur (38) est placée en aval de ladite au moins une chambre d'exposition (2).

15. Procédé d'exploitation d'une installation d'exposition (1) pour la gazéification en continu d'au moins une culture cellulaire (40) dans au moins un logement d'échantillon (41) avec au moins un gaz d'essai, notamment selon l'une des revendications 1 à 14,
- dans lequel, dans au moins un dispositif de conditionnement (3), ledit au moins un gaz d'essai pour la gazéification de ladite au moins une culture cellulaire (40) est conditionné,
- dans lequel, dans ledit au moins un dispositif de conditionnement (3), notamment dans au moins un humidificateur de gaz d'essai (23), l'eau à doser, provenant d'au moins un réservoir d'eau (25) pressurisé, est acheminée, à une pression prédéterminée, dans un capillaire d'eau (29) et l'eau est évaporée après avoir quitté le capillaire d'eau (29) et/ou
- dans lequel, dans ledit au moins un dispositif de conditionnement (3), notamment dans au moins une addition de composant d'essai (4), le standard (5) à doser, est acheminé, à partir d'au moins un réservoir de standard (6) pressurisé à une pression prédéterminée, vers un capillaire de liquide (11) et le standard (5) est évaporé après avoir quitté le capillaire de liquide (11).

16. Procédé selon la revendication 15,
- dans lequel la pression, dans au moins un réservoir d'eau (25) et/ou un réservoir de standard (6), est réglée de préférence par l'intermédiaire d'au moins un raccordement au gaz comprimé (8) et/ou d'au moins une soupape de réduction (27), et
- dans lequel, de préférence, la pression dans le réservoir d'eau (25) et/ou dans le réservoir de standard (6) est réglée, de manière constante, à une valeur comprise entre 1 bar et 4 bars, de préférence entre 1,1 bar et 3 bars, notamment entre 1,2 bar et 2 bars, plus particulièrement entre 1,3 bar et 1,5 bar.

17. Procédé selon la revendication 15 ou 16,
- dans lequel la température de l'eau évaporée et/ou du standard évaporé (5) dans l'évaporateur (12, 30) est réglée à une température comprise entre 100 °C et 300 °C, de préférence entre 120 °C et 200 °C, notamment entre 130°C et 170°C, et/ou
- dans lequel la pression de l'eau évaporée en amont du capillaire de vapeur d'eau (13) et/ou la pression du standard évaporé (5) en amont du capillaire de vapeur (31) est réglée à une pression comprise entre 1 bar et 4 bars, de préférence entre 1,1 bar et 3 bars, notamment entre 1,2 bar et 2 bars, plus particulièrement entre 1,3 bar et 1,5 bar.

18. Procédé selon l'une des revendications 15 à 17,
dans lequel l'eau évaporée, le standard évaporé (5) et/ou le gaz porteur, en particulier pour former le gaz d'essai, en aval de l'évaporateur (30) de l'eau, de l'évaporateur (12) de standard, du capillaire de vapeur d'eau (31) et/ou du capillaire de vapeur (13), sont mélangés dans au moins un mélangeur (32), en particulier un mélangeur statique (32).

19. Procédé selon l'une des revendications 15 à 18,
- dans lequel, dans ledit au moins un dispositif de conditionnement (3), un débit massique prédéterminé d'air et/ou de dioxyde de carbone (CO2) est acheminé, notamment mélangé, et
- dans lequel, de préférence, du dioxyde de carbone est acheminé avec un débit massique déterminé compris entre 5 Nl/h et 100 Nl/h, de préférence entre 10 Nl/h et 50 Nl/h, en particulier entre 15 Nl/h et 30 Nl/h, et/ou de l'air est acheminé avec un débit massique déterminé compris entre 100 Nl/h et 1 200 Nl/h, de préférence entre 200 Nl/h et 800 Nl/h, notamment entre 300 Nl/h et 500 Nl/h.

20. Procédé selon la revendication 19,
- dans lequel ledit au moins un débit massique est réglé par au moins un régulateur de débit massique (19, 20) et
- dans lequel, de préférence, le gaz traverse un dispositif répartiteur (22) en aval d'un régulateur de débit massique (19, 20).

21. Procédé selon l'une des revendications 15 à 20,
- dans lequel ledit au moins un logement d'échantillon (41) est tenu prêt pendant la gazéification de la culture cellulaire (40) correspondante dans une chambre d'exposition (2) conditionnée thermiquement, de préférence à une température déterminée entre 34 °C et 38 °C, de préférence entre 35 °C et 37 °C, notamment au moins essentiellement à 36 °C, et/ou
- dans lequel le gaz d'essai est conditionné thermiquement, en particulier refroidi, à une température déterminée comprise entre 36 °C et 40 °C, de préférence entre 37 °C et 39 °C, notamment d'au moins essentiellement 38°C, immédiatement avant d'être acheminé vers une boîte cellulaire (34) agencée dans la chambre d'exposition (2).

22. Procédé selon la revendication 21,
- dans lequel au moins une température du gaz d'essai dans la boîte cellulaire (34) est détectée et, de préférence, réglée, et
- dans lequel, de préférence, ladite au moins une température du gaz d'essai dans la boîte cellulaire (34) est réglée à une température déterminée entre 35 °C et 39 °C, de préférence entre 36 °C et 38 °C, notamment à 37 °C (± 0,5 °C).

23. Procédé selon la revendication 21 ou 22,
- dans lequel le gaz d'essai dans la boîte cellulaire (34) traverse, côté entrée et/ou côté sortie, un diffuseur (42) s'élargissant et/ou un diffuseur (45) se rétrécissant et/ou
- dans lequel le gaz d'essai dans la boîte cellulaire (34) traverse partiellement au moins un treillis (44) et/ou une tôle perforée au-dessus dudit au moins un logement d'échantillon (41) et/ou
- dans lequel le gaz d'essai dans la boîte cellulaire (34) traverse, côté entrée, au moins un treillis (43) et/ou une tôle perforée.

24. Procédé selon l'une des revendications 15 à 23,
- dans lequel le gaz quittant ladite au moins une chambre d'exposition (2) est conduit, notamment partiellement, par une conduite pour les prises d'échantillons conditionnée thermiquement, à un dispositif d'analyse (37) pour analyser la composition du gaz et/ou
- dans lequel le gaz quittant ladite au moins une chambre d'exposition (2) est conduit, au moins partiellement, par une conduite de condensation, de préférence conditionnée thermiquement, à un condenseur (38) pour condenser les parties du gaz à l'état de vapeur.
